# EUROPEAN PATENT APPLICATION

(11) **EP 3 808 319 A1**
(43) Date of publication of application: **21.04.2021**
(21) Application number: 20201672.1
(22) Date of filing: 14.10.2020
(51) Int. Cl.: A61F 7/00, A61B 18/04, A61B 18/12, A61B 18/18, A61F 7/02

(54) **WEARABLE MEDICAL, PHYSIOTHERAPEUTIC AND/OR PROTECTIVE DEVICE**

(30) Priority: 15.10.2019 IT 201900018770
(71) Applicant: Green Working for the Development of the World S.r.l. - in Sigla GWDW, 37057 San Giovanni Lupatoto (IT)
(72) Inventor: D'AMICO, Piermatteo, 37127 Verona (IT); BOSIO, Giulia, 37023 Grezzana (IT)
(74) Representative: Contadin, Giorgio

(57) **Abstract**

A wearable medical, physiotherapeutic and/or protective device (1; 50; 100; 150) comprising a laminar body (2) which normally, in conditions of non-use of the medical, physiotherapeutic and/or protective device (1; 50; 100; 150), assumes a substantially flat shape and which, at least in conditions of use of the medical, physiotherapeutic and/or protective device (1; 50; 100; 150) coinciding with the situation in which the medical, physiotherapeutic and/or protective device (1; 50; 100; 150) is worn by a patient, it assumes a shape which follows or adapts to a localized anatomical area, to be subjected to heat therapy, of the human body of said patient, and heating means (3) coupled to the laminar body (2) and adapted to be activated when the laminar body (2) is worn by the patient to act on the localized anatomical area of the human body to be subjected to such heat therapy. In detail, the laminar body (2) consists of an elastic containment exoskeleton (4) in which a housing pocket (5) is identified which houses a flexible film (6) emitting electromagnetic radiation, forming the heating means (3) and electrically connected to an electric power supply source (7), adapted to be placed close to the localized anatomical area to be subjected to heat therapy.

## Description

The present invention relates to a wearable medical, physiotherapeutic and/or protective device, especially suitable for localized applications of heat therapy (more precisely, of hyperthermia), to support, by way of purely explanatory and indicative example, precision medicine, physiological medicine, immune-stimulating therapies, aesthetic-functional treatments, human medicine in general and veterinary medicine in particular.

### BACKGROUND ART

As known, heat therapy involves the use of heat as a therapeutic tool for the treatment of diseases, such as tumors, or for the restoration of optimal physiological conditions. Generally, in oncological therapy, heat is applied with the aim of increasing the temperature of the diseased tissue only by a few degrees, in order to exploit the induced increase in the tumors' sensitivity to ionizing radiation and to some medications. The heat therapy treatment in question, where the temperature range at which it occurs is substantially between 41°C and 47°C, is called "hyperthermia".

At these temperatures, tumors' greater sensitivity to heat compared to healthy tissues has been experimentally observed, as the tumor cells don't have a very cohesive surface and the heat transmitted thereto contributes to their damage and to the better efficacy of the drugs.

Currently, although only in the context of reducing pain from arthrosis or trauma suffered by people (and therefore not in the treatment of invasive diseases such as oncological ones), wearable type braces are used which, through resistive circuits or heating elements by chemical reaction (such as specific patches), heat the localized anatomical area of the human body (to be subjected to heat therapy).

The primary advantage of these medical, physiotherapeutic and/or protective devices of a known type lies in the fact that the concrete possibility of the patient wearing them independently and more or less easily allows the patient to keep the painful part warm and to perform therapeutic heat treatments on a more continuous basis, therefore not only within hospitals and not only periodically, at distinct and pre-established (albeit regular) moments of a certain time frame, so as to provide continuity to countering pain in the intervals between one hospital or specialist session and another.

However, these wearable containment braces have some drawbacks, one of which lies in the fact that, during the use thereof - moreover specific and limited to the reduction of pain from arthrosis or trauma -, the fabric with which it is made becomes impregnated with sweat, and over time, with unhealthy bacterial loads.

It disadvantageously follows that the part of the human patient's body which is thereby wetted is subject to sudden cooling, especially in the winter season, producing considerable trauma and/or pain in the muscles.

Another drawback of the heating braces of the prior art, which may be worn at the localized anatomical area of the human body to be subjected to heat therapy, is related to the bad odor developed by sweat during use: undoubtedly, this does not allow a comfortable use of this type of known brace.

An additional negative aspect is that the dimensions of the wearable heating braces of the prior art are such as to make them not very comfortable to wear under clothing, regardless of the type of the latter.

However, most importantly, the wearable heating braces available on the market today have the drawback that the standard and rigid shapes thereof do not allow optimal adherence to the various shapes and sizes of the localized anatomical area of the human body which must house them for the treatment of illness and/or pain.

An example of a brace or heating apparatus of therapeutic type, removably applicable to a part of a patient's body (such as a wrist) to be subjected to heat therapy, with a similar relevant problem is disclosed by the prior art document published as US2008/0262393 A1.

Indeed, the therapeutic radiant heating apparatus shown in this known document - having for example the shape of a pad, a block, a garment, a blanket, a shoe, a hat, a bed, a pillow and so on - comprises an outer fabric casing or covering (reference numeral 13) made of cotton, artificial or natural fiber, and one or more heating elements (reference numeral 25), for example in the form of tiles or layers of glass fiber (reference numeral 37), placed inside respective inner pockets (reference numeral 70 or 216) each having an opening (reference numeral 219) closed by fastening elements (reference numeral 222). More specifically, as can be deduced from a complete reading of paragraphs 28 and 29 of document US2008/0262393 A1, the glass fiber of the radiant tiles may be, in an embodiment, impregnated and saturated with a chemical compound which may be a mixture of low and high-strength carbon to provide mechanical strength.

Document US2008/0262393 A1 further explains that:
- the materials useable for the heating elements may also be different from glass fiber;
- the materials forming the heating elements and providing mechanical strength are basically made of carbon;
- the heating elements may be a radiant black block which may have a high efficiency in terms of radiant heat dispersion.

It is therefore apparent that the heating elements housed inside the outer casing of the apparatus of document US2008/0262393 A1 are rigid bodies or at least not at all flexible, and in return, they are not anatomically adaptable or deformable, but simply resting on the area of the person's body, such as the wrist, to be therapeutically heated by means of heat (to a maximum temperature of 54°C).

This inevitably and negatively involves for US2008/0262393 A1, on the one hand, a reduction in the effectiveness, in the unit of time, of the therapeutic heat treatment on the area of the person's body, or in other words, a lower operating efficiency given by the need to apply more electrical power to obtain the same effectiveness in the unit of time, and on the other hand, the need to change the position of the device on the affected area of the person's body even several times to obtain a better therapeutic heat treatment, however incomplete in absolute terms and optimizable.

To this is added, negatively, even the fact that the apparatus shown in US2008/0262393 A1, when in use, is not very comfortable to wear, it still has a certain bulk and the impacts possibly suffered by it at the heating elements, due to the constructive conception of the latter, expose them to harmful damage risks, however slight and partial.

It should also not be overlooked that in the current state of the art it is substantially impossible or at least problematic to create personalized or customized wearable heating braces, usable for different anatomical areas of the human body, in industrial mode. There are other types of wearable medical and/or physiotherapeutic devices on the market, such as bands made mainly of a uniform and monolithic fabric of neoprene or similar materials: even these devices of the prior art are therefore not very breathable and unhygienic in conditions of use.

### PURPOSES OF THE INVENTION

The present invention intends to overcome the drawbacks of the prior art which have just been briefly highlighted.

In particular, a general purpose of the invention is to provide a wearable medical, physiotherapeutic and/or protective device which offers continuity in the fight against a specific disease, such as that of tumor cells, even during the interval, generally inoperative or inactive, between a hospital application and another, or between a specialized medical treatment and another, performed for example by means of chemotherapy and/or radiotherapy, for the treatment of a disease, for example oncological, and makes pharmacological therapies more effective.

In other words, purpose of the invention is to indicate a wearable medical, physiotherapeutic and/or protective device which allows to perform a heat therapy, generally of hyperthermia, a greater number of times in a given time period than that associated with a traditional and common course of medical/hospital treatment, for example chemotherapy and/or radiotherapy.

Within these purposes, a first task of the present invention is to develop a wearable medical, physiotherapeutic and/or protective device which, precisely because it can be worn anywhere and at any time (at home, during work and/or free time) by a patient, improves the quality of life thereof, freeing them from the need to access specialized centers, such as the cancer wards of a hospital, which are always psychologically difficult to deal with and still create discomfort and states of upset and/or anxiety.

A second purpose of the invention is to devise a wearable medical, physiotherapeutic and/or protective device which, in conditions of use, assumes an anatomical shape with respect to the area of the user's body to which it is applied and which therefore adapts in an almost perfect manner to the shapes and sizes of any localized anatomical area of the human body to be treated by means of heat therapy (or thermotherapy) to treat a disease and/or pain better and more effectively than the known equivalent devices.

In other words, the invention presented herein proposes to bring a decisive improvement compared to the equivalent devices of the prior art, consisting for instance in US2008/0262393.

Another purpose of the invention is to provide a wearable medical, physiotherapeutic and/or protective device which, compared to similar devices of the prior art, is more effective in helping to treat diseases, including invasive ones, such as for example a tumor and/or rheumatic and arthritic pains as well as helping to contain the formation of sores and/or wounds on the patient's skin, the reactivation of peripheral vascularization and athletic preparation and cool-down.

Within the scope of the latter purpose, it is the task of the invention to provide a wearable medical, physiotherapeutic and/or protective device, unlike similar devices already existing on the market, whether only for comfort or to help the patient counteract the disease or physical pain suffered, such that the use thereof is not in some manners counterproductive, in the sense that it does not negatively induce further physical problems in the human patient's body.

A further purpose of the present invention is to provide a wearable medical, physiotherapeutic and/or protective device which, especially in conditions of use (i.e., worn by a patient), is more hygienic and above all more comfortable than the devices of the prior art directly comparable thereto, of which the apparatus shown in US2008/0262393 A1 is an example.

An additional purpose of the invention is to create a wearable medical, physiotherapeutic and/or protective device which is producible on a large scale and industrial level quickly and at low costs, lower than those incurred to produce the known wearable heating devices, thereby allowing the use thereof on various distinct parts of the human body of one or more patients.

Another purpose of the invention is to indicate a wearable medical, physiotherapeutic and/or protective device, which is more practical to use, effectively and efficiently, than similar known devices, such as that shown in document US2008/0262393 A1.

An additional purpose of the present invention is to provide a wearable medical, physiotherapeutic and/or protective device which has a smaller bulk than the known thermotherapeutic heating devices, such as for example the one referred to in prior document US2008/0262393 A1, both in conditions of use and in conditions of non-use.

A last but not least purpose of the invention is to provide a wearable medical, physiotherapeutic and/or protective device that can be worn under any cloth in a more comfortable, more practical, less invasive and less evident manner from the outside compared to similar devices of the prior art, shown for instance in US2008/0262393 A1. Said purposes are achieved through a wearable medical, physiotherapeutic and/or protective device as in the appended claim 1, to which reference is made for the sake of convenient presentation.

Further detailed technical features of the wearable medical, physiotherapeutic and/or protective device of the invention are indicated in the respective dependent claims.

The aforesaid claims, hereinafter specifically and concretely defined, are intended as an integral part of the present description.

### ADVANTAGES

Advantageously, the wearable medical, physiotherapeutic, radiant, thermotherapeutic and/or protective device of the invention acts as an aid in the treatment of diseases, including invasive ones, such as for example a tumor, and/or rheumatic and arthritic pain, in a more effective manner compared to wearable devices, provided with heating means, of current similar use.

This is due to the fact that the laminar body of the wearable medical, physiotherapeutic and/or protective device claimed herein essentially consists of an elastic, soft, easily transportable, containment exoskeleton which can be positioned on the human patient's body, in which a housing pocket is identified. which receives a flexible film emitting electromagnetic radiation, preferably a long infrared radiation, forming the heating or radiating means and adapted to be placed close to the localized anatomical area to be subjected to heat therapy (hyperthermia or thermotherapy) of the human patient's body and electrically connected to an electric power supply source.

Equally advantageously, the punctual and localized thermotherapy proposed by the wearable heating device of the invention is carried out by means of elements emitting an electromagnetic radiation, preferably a long infrared radiation in class "C" (defined as vital frequency, with a value in the interval 4÷20µm), which stimulates the blood circulation, muscles and skin of the human body of a patient, a person or an athlete and contributes to the recovery of the optimal physiological condition of these subjects.

The wearable medical, physiotherapeutic and/or protective device of the invention therefore allows to reactivate the blood circulation and to restore the peripheral vascularization, being effective both for rheumatic and arthritic pains as well as for athletic and muscular preparation or cool-down at a sports or military level.

Still advantageously, due to the elastically yielding structure also in the radiant film, and more particularly and more preferably, to the weft of the reticulated body belonging thereto, the wearable medical, physiotherapeutic and/or protective device of the invention is capable of supporting the patient's body more efficiently than the equivalent known devices (shown for example in US2008/0262393 A1), with respect to which, when worn by the patient at the area to be subjected to heat therapy, is anatomical and clearly remains more adherent to the skin and to the tendon and muscle tissues of the human body. Equally, advantageously the wearable thermotherapeutic, medical, physiotherapeutic and/or protective device of the present invention is producible in a serial manner in little time, for example by means of a 3D printer, unlike what occurs for the devices of the prior art comparable thereto.

Advantageously, therefore, the wearable thermotherapeutic, medical, physiotherapeutic and/or protective device of the invention has a considerably more effective production cost than that of the similar known devices, with the same cost parameter of the labor involved in the calculation of such cost.

Advantageously, moreover, again due to the completely elastically yielding structure thereof (the main components thereof, i.e., the containment exoskeleton and the radiant film are indeed flexible), the wearable thermotherapeutic, medical, physiotherapeutic and/or protective device of the invention has a smaller bulk than the known devices both in conditions of use and in conditions of non-use, with respect to which it can also be used in a more practical and more comfortable manner, as well as more effective since it anatomically wraps the area of the body of the person undergoing therapy. Advantageously, moreover, through the invention it is possible to create wearable medical, physiotherapeutic and/or protective devices which allow thermotherapy, or more specifically, localized hyperthermia at the optimal temperature for each of the pathological conditions to be faced and for each different sensitivity of the patient to be treated.

Again advantageously, the wearable medical, physiotherapeutic and/or protective device of the invention has a simpler constructive concept than that of the closest prior art document, published with US2008/0262393 A1, since, contrary to what is necessarily included in this prior document, it does not comprise any stiffening member (or resilient reinforcement, reference numeral 61) which surrounds the housing pockets of the radiant elements, prevents the latter from bending and is coupled to the outer casing.

Therefore, in the prior document US2008/0262393 A1 a component is necessarily provided which considerably limits the flexibility of the claimed device, and therefore inhibits the possibility, when worn by the user, of anatomically following the same user's body area to be subjected to therapy by means of radiant heat, especially if such area is located at a joint of the human body, to the detriment of the effectiveness of the thermotherapeutic treatment: the apparatus described in US2008/0262393 A1 therefore teaches in the direction substantially opposite to that which the wearable medical, physiotherapeutic and/or protective device of the invention is mainly oriented.

Not surprisingly, the device described in US2008/0262393 A1 also includes that, in order to be stably - albeit removably - coupled to the person's body, at the area affected by pain and disease and to be thermally treated, the outer casing (reference numeral 13) made of cotton, artificial or natural fiber is provided with one or more straps (reference numeral 240) with couplable ends (for example with Velcro®) which are wrapped around the human body and are in no manner included in the wearable radiant, medical, physiotherapeutic and/or protective device of the invention.

### BRIEF DESCRIPTION OF THE DRAWINGS

Further features and particularities of the present invention will become more evident from the following description, relating to preferred embodiments of the wearable medical, physiotherapeutic and/or protective device of the invention itself, provided for indicative and illustrative non-limiting purposes, with reference to the accompanying drawings in which:
- figure 1 is an exploded assonometric view of a first embodiment of the wearable medical, physiotherapeutic and/or protective device of the invention;
- figure 2 is a front view of a constructional assembly of the device of figure 1;
- figure 3a is a first enlarged assonometric view of a constructional detail of figure 1;
- figure 3b is a second enlarged assonometric view of the detail of figure 3a;
- Figure 4 is an assonometric view of a constructional detail of figure 2;
- figures 5-7 are three diagrammatic assonometric views of three distinct embodiments of the wearable medical, physiotherapeutic and/or protective device of figure 1.

### DETAILED DESCRIPTION OF A PREFERRED EMBODIMENT OF THE INVENTION

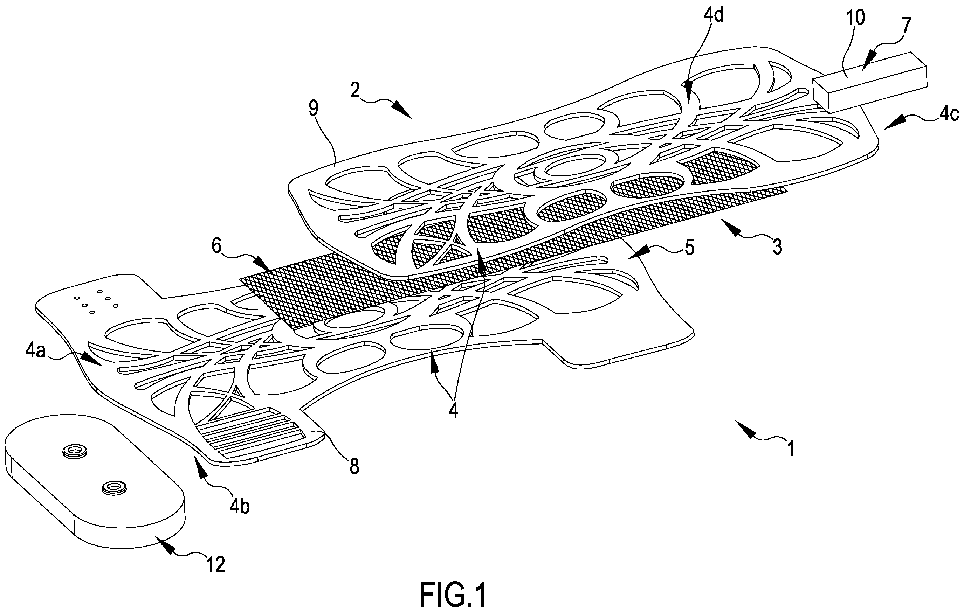

The wearable medical, physiotherapeutic and/or protective device of the invention is illustrated in figure 1, where it is globally indicated with 1.

As can be seen, the wearable medical, physiotherapeutic, radiant, thermotherapeutic and/or protective device 1 comprises:
- a laminar body 2 which normally, in conditions of non-use of the medical, physiotherapeutic and/or protective device 1, assumes a substantially flat shape and which, at least in conditions of use of the medical, physiotherapeutic and/or protective device 1 coinciding with the situation in which said medical, physiotherapeutic and/or protective device is worn by a patient, it assumes a shape which follows or adapts to a localized anatomical area, to be subjected to heat therapy (thermotherapy or hyperthermia), of the human body of said patient;
- heating means, indicated as a whole with 3, coupled to the laminar body 2 and adapted to be activated at any time and in any place, generally by the patient or by a companion, when the laminar body 2 is worn by the same patient to act on the localized anatomical area of the human body to be subjected to heat therapy.

Basically, according to the invention, the laminar body 2 of the wearable medical, physiotherapeutic and/or protective device 1 consists of an elastic containment exoskeleton 4 in which is identified (or defined) a housing pocket 5 which, in turn, receives an elastic film 6 emitting electromagnetic radiation, consisting of or forming the heating means 3 and electrically connected to an electric power supply source, indicated with the reference numeral 7, adapted to be directly and effectively arranged close to the localized anatomical area, also including joints, of the human body to be subjected to heat therapy. Particularly but only preferably, the flexible radiant film 6 emitting electromagnetic radiation is removably coupled to the elastic containment exoskeleton 4.

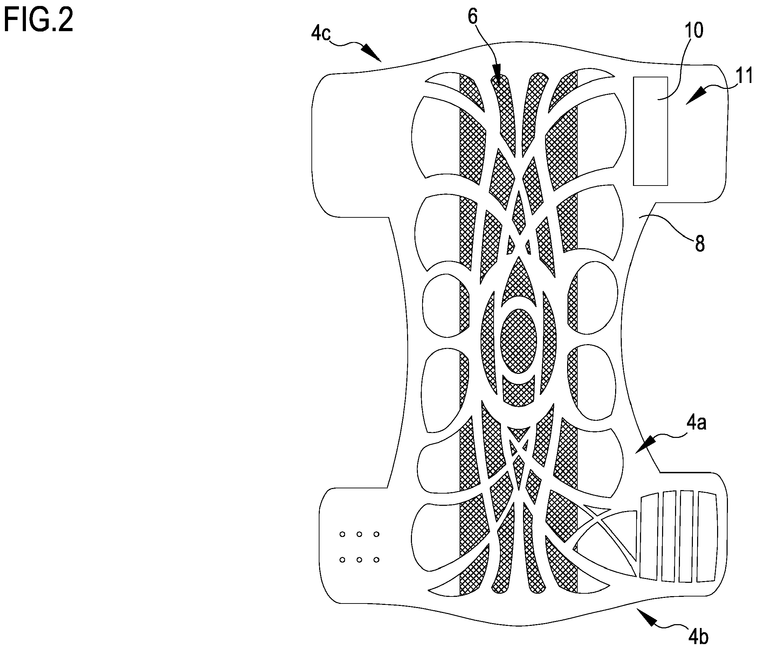

Furthermore, as can also be seen in figure 2, the flexible radiant film 6 emitting electromagnetic radiation is arranged in the central part 4a of the elastic containment exoskeleton 4, since the housing pocket 5 itself is obtained in such central part 4a of the elastic exoskeleton 4.

Preferably but not necessarily, the flexible radiant film 6 emitting electromagnetic radiation emits infrared radiation.

Even more preferably, although not limitedly, the infrared thermal radiations emitted by the radiant, flexible and emitting film 6 are of the long type in class C (also defined as biogenic rays), have a frequency in the range 2÷30 µm, more preferably in the range 4÷14 µm and induce, in the treated part, temperatures from 30 to 45°C sufficient for applications both in general medicine (arthrosis, vascularization and trauma) and in precision medicine (oncological applications).

In addition to what has already been pointed out, the long infrared radiation, in class C, applied by means of the wearable medical, physiotherapeutic and/or protective device 1 of the invention, is particularly effective in containing and reducing pressure sores as well. As further apparent from specialized studies carried out in oncology, long infrared radiation, in class C, is therefore an excellent aid in supporting the treatment of tumors. The properties thereof and the temperature produced thereby in living organisms allow a better efficacy of the medical treatments performed by means of the wearable medical, physiotherapeutic and/or protective device 1 of the invention, compared to those of other known equivalent devices.

In addition, the frequency of the infrared radiation produced, preferably, by the flexible film 6 emitting electromagnetic radiation associated with the wearable radiant, medical, physiotherapeutic and/or protective device 1 of the invention is an effective stimulator for the natural production of collagen and for skin regeneration aimed at the aesthetic-functional restoration of the skin.

Even for animals, the applications of the wearable radiant, medical, physiotherapeutic and/or protective device 1 of the invention which exploit an electromagnetic radiation emitting film of the infrared type may be in both precision medicine and physiotherapy. The mechanisms of action of the infrared radiation preferably produced by the wearable medical, physiotherapeutic and/or protective device 1 of the invention are mediated by a direct proapoptotic effect on tumor cells, in synergy with the chemotherapy and/or radiotherapy cycle, immunomodulating and immunostimulating, analgesic through the release of endorphins and direct action on the nociceptive endings.

It should be noted that the adjective "proapoptotic" is generally used in reference to a gene, the expression product of which consists of proteins which make cells more susceptible to apoptosis, a term which indicates a form of programmed cell death. Furthermore, preferably but not bindingly, the flexible film 6 emitting electromagnetic radiation comprises a hybrid printed electric circuit (not visible in the attached figures), made partially of a first electrically conductive material and partially of a second electrically insulating material, so that said printed electric circuit is made of an overall material (set of said first material and said second material) which can be defined as a semiconductor.

Preferably, the electric circuit is printed using high-quality inks in silver (first electrically conductive material above) and carbon (second electrically insulating material above). This electrical circuit is designed to operate at very low voltage (max 9 Volts, as will be better highlighted shortly), it does not become incandescent and does not cause burns or other problems for the user.

Many therapeutic devices on the market used for the localized heating of a person's body include, as electrical circuits, combined carbon (black body) and copper (electric power supply) systems having significant voltages and amperages (68-110- 220 Volt) and therefore rather dangerous for the user; moreover, the coexistence of copper and carbon in the same electrical circuit may induce sparking (cause of ignition of fire).

The flexible film 6 of the heating means 3 of the wearable medical, physiotherapeutic and/or protective device 1 of the invention preferably uses silver, a nobler and more stable element than copper and which does not induce any sparks in contact with carbon (in turn essential for the emission of infrared rays) which also has remarkable antibacterial properties.

The innovative printing technology provided, in the invention, for the flexible film 6 allows to obtain a high-precision semiconductor system, so as to allow, for the first time, to calibrate the absorbed electric power, the emitted thermal power and the exact frequency of infrared rays necessary to create a precise thermotherapy for each different anatomical part of the body and for each different user.

For the wearable medical, physiotherapeutic and/or protective device 1 of the invention, temperature and frequency ranges have been defined for each part of the body (based on parameters such as: skin thickness, presence of important blood vessels, user age), some examples of which are given below:

| **PARTS OF THE BODY** | **TEMPERATURE** | **INFRARED RAY FREQUENCY (µm)** |
|---|---|---|
| KNEE | 34÷38 °C | 10 |
| HAND | 32÷36 °C | 8 |
| BACK | 36÷40 °C | 12 |
| QUADRICEPS FEMORIS | 36÷40 °C | 9 |

By means of the wearable medical, physiotherapeutic and/or protective device 1 of the invention, it is however possible to adjust the temperature, applied to the anatomical area of the body to be treated, according to the different heat-skin sensitivities, maintaining the ideal frequency for this anatomical part.

In an advantageous but not binding manner, the elastic containment exoskeleton 4 is made of printed polymeric material for medical use, for example by means of a three-dimensional printer.

This polymeric material is any of the hypoallergenic plastic materials selected from the group consisting of synthetic polyamide (such as nylon), carbon, graphene, silicone, copolyester, acrylonitrile-styrene-acrylic ester, polypropylene, polytetrafluoroethylene (also known by the acronym PTFE or the trade name Teflon), thermoplastic polymer such as acrylonitrile-butadiene-styrene (also known by the acronym ABS) or polyethylene terephthalate (also known by the acronym PET G) and/or other similar polymers: the elastomeric feature of the weft of the elastic containment exoskeleton 4 allows the wearable medical, physiotherapeutic and/or protective device 1 of the invention to conform perfectly to the human body housing it and to support it more effectively than the equivalent devices available on the market (even those of the tensioned type).

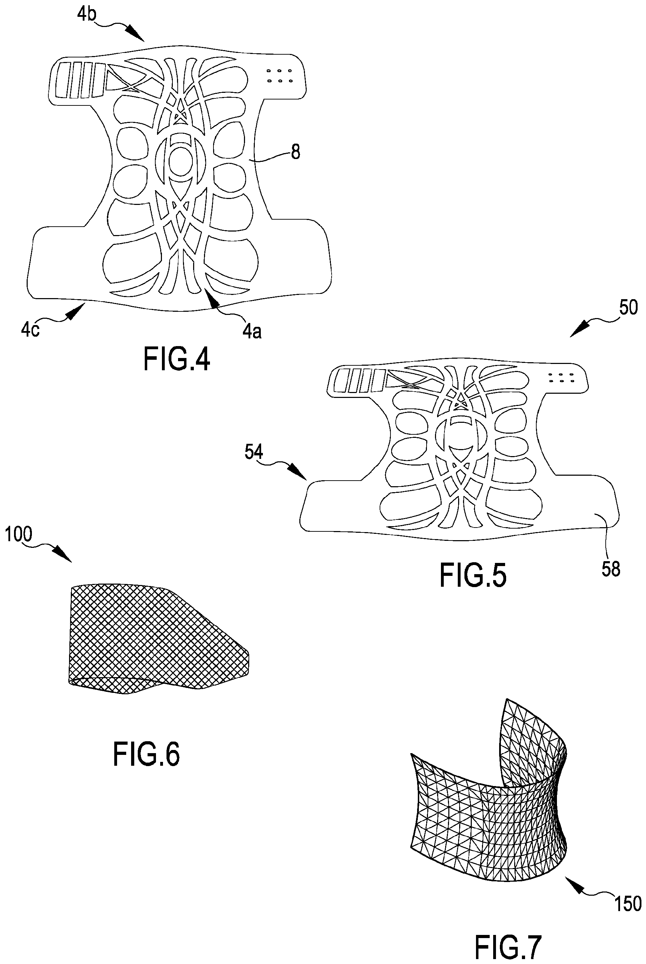

Preferably, the elastic containment exoskeleton 4 is adapted to be used on the patient's back, and depending on the intended use and the human body on which the heat therapy treatment is to be performed, and has a thickness in the range 2.5÷5 mm; in the preferred but not exclusive case in which the elastic containment exoskeleton 4 is obtained by means of a 3D printer, the filament with which it is made (for example in plastic material such as PET G, a particularly versatile filament, with very high mechanical performance and easy to print) is non-absorbent, is hypoallergenic and has a thickness of 1÷2 mm. According to the preferred embodiment of the invention described herein, the elastic containment exoskeleton 4 comprises a base reticular structure 8 (visible independently in figure 4) and a reticular covering structure 9, both visible in figure 1, stably coupled to each other through joining means, not illustrated and comprising, for example, a welding section by thermoforming, so as to define a free peripheral section, not indicated and defined on one of the two shorter sides 4b, 4c of the elastic containment exoskeleton 4, in which an access mouth is identified, also not indicated, which communicates with the housing pocket 5 and through which the electromagnetic radiation emitting film 6 is introduced into the housing pocket 5 itself.

The combination of the plastic material with the perforated mesh structure makes the elastic containment exoskeleton 4 somewhat deformable - and therefore effectively and easily adaptable to various shapes in localized areas of the human body of the patient to be treated by means of thermotherapy, resulting in being well adherent - and at the same time, quite resistant from a mechanical point of view, as well as breathable, easy to clean and sanitize, extraneous to the absorption of sweat and the emanation of odors, and therefore hygienic: these favorable technical properties are not found simultaneously, in a single item, in the equivalent prior art (consider the aforementioned neoprene devices).

In essence, therefore, the elastic exoskeleton 4 is anatomically shaped both as a lateral profile and as a graphic design, so as to follow, in the best possible manner and almost faithfully, the muscle fascia of the localized anatomical area of the human body to be subjected to heat therapy, usually hyperthermia.

As regards the electric power supply source 7, figures 1 and 2 show that it comprises a power supply battery 10 housed in a surface seat 11 open outwards, obtained in the outer surface 4d of the elastic containment exoskeleton 4, in this case in particular the reticular covering structure 9.

In more detail, the electric power supply source 7 comprises a battery 10 consisting of a plurality of mini-accumulators, of the type known per se to those skilled in the art, having an electrical voltage in the range 5÷9 Volts and an electric current with a value not exceeding 5 ampere hours (Ah).

The values of the electrical voltage applied by the wearable medical, physiotherapeutic and/or protective device 1 of the invention are therefore significantly lower than those indicated in the document US2008/0262393 A1 which indicates 12 Volts as a minimum value but mainly describes variants with 110, 220 and 240 Volts, decidedly more invasive in the treatment step on the person's body.

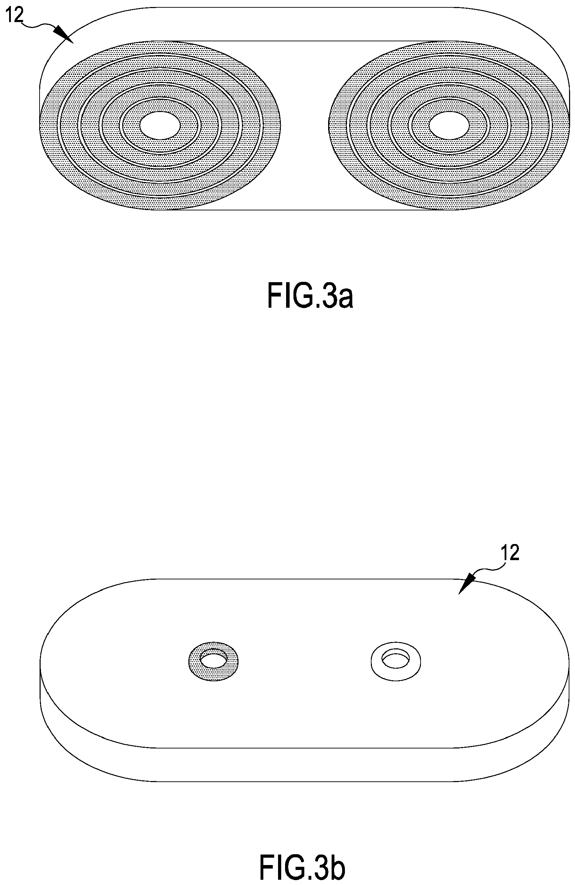

Furthermore, the electric power supply source 7 preferably also includes an electric mains power supply, not illustrated for simplicity of presentation, used for recharging the battery 10 or for the direct power supply of the flexible film 6 emitting electromagnetic radiation. Advantageously, preferably but not limitingly, the heating means 3 also comprise a radiofrequency emitting plate or head 12 (frequencies between 300 KHz and 300 MHz, corresponding to wavelengths between 1m and 1m, typically having a value of 13.56 MHz and a power of 200 Watt), still visible in figures 1 and 2 but in greater detail, independently, in the details of figure 3a and figure 3b, adapted to be placed close to the localized anatomical area of the human patient's body to be subjected to heat therapy (hyperthermia or thermotherapy): the radiofrequency emitting plate 12 is coupled to the elastic containment exoskeleton 4 and is also electrically connected to the electric power supply source 7.

The combined use of the flexible film 6 emitting electromagnetic radiation and of the radio frequency emitting plate or head 12 allows in particular to:
- bring the therapeutic temperature deeper into the patient's body;
- damage tumor cells even more effectively and reactivate healthy cells;
- restore the lymphatic vascularization in loco, thus allowing medical therapies (for example chemotherapy or radiotherapy treatments) to reach the diseased cells (for example tumor cells) more effectively, increasing the pharmacological efficacy thereof and in return reducing the duration thereof;
- restore the best physiological condition in the part of the human body damaged by the disease, such as a tumor, trauma or physiological degeneration;
- reduce loco-regional pain, in a more pointed and more precise manner with respect to the known devices.

The infrared frequency combined with the localized radiofrequency, emitted by the wearable medical, physiotherapeutic and/or protective device 1 of the invention, are directed towards the localized anatomical area of the human patient's body to be treated, with the aim of allowing patients to perform thermotherapy continuously and not only in hospitals or specialized medical centers, but also in any place and at any time of daily life. By doing so, it is possible to give continuity to the fight against diseased cells, for example and typically tumor cells, in the intervals between one treatment cycle and another (for example between one chemotherapy and/or radiotherapy cycle and the next).

For example, the radiofrequency allows to raise the temperature of tumor cells and bring them to apoptosis, revascularize the lymphatic vessels to allow chemotherapy to reach the tumor cells, while infrared radiation allows peripheral lymphatic revascularization, stimulation of the patient's immune system, the interruption of the transcription of tumor cells in the DNA and the inhibition of the reproduction and proliferation thereof, as well as the improvement of the quality of the patient's psycho-physical condition.

It is understood that, in other optional variants of the wearable medical, physiotherapeutic and/or protective device of the invention, not shown below, the heating means may comprise more than one radiofrequency emitting plate or head, always suitably coupled to the elastic containment exoskeleton, and more specifically, the covering mesh of the latter.

Also in this case, preferably but not exclusively, the radiofrequency emitting plate or head 12 is removably coupled to the elastic containment exoskeleton 4 through snap coupling means, not shown for simplicity of presentation, with consequent ease of replacement of this component.

Conveniently but not exclusively, the wearable medical, physiotherapeutic and/or protective device 1 of the invention also comprises a pair of operating sensors, not illustrated for simplicity of presentation, coupled to the elastic containment exoskeleton 4 and electrically connected to the electric power supply source 7, adapted to detect the vital values of the human patient's body while, for example, he or she is using the wearable medical, physiotherapeutic and/or protective device 1 itself.

Furthermore, the wearable medical, physiotherapeutic and/or protective device 1 of the invention includes a central processing and control unit, electrically connected to the electric power supply source 7, useful for managing not only (and at least) the operating parameters of the heating means 3, following commands transmitted by a user (the same patient, a companion or a health care worker), but also of the operating sensors just mentioned and storing the vital data of the patient the localized anatomical area of the human body of which is subjected to the adopted heat therapy.

Figures 5-7 show three assonometric views of as many possible and distinct, but not limiting, embodiments of the wearable medical, physiotherapeutic and/or protective device of the invention, indicated respectively with 50, 100 and 150, useable to be applied at a patient's knee, mouth or hand, respectively.

Actually, figure 5 properly shows only the base mesh 58 of the elastic exoskeleton 54 of the wearable medical, physiotherapeutic and/or protective device 50 of the invention.

From an operational point of view, for example, in the thermal radiofrequency ablation achievable by means of the wearable medical, physiotherapeutic and/or protective device 1 of the invention, a high-frequency alternating current, with electrical power contained at 150 Watts, creates temperatures between 50°C and 70°C.

The frequency is emitted by the radiant flexible film 6 contained in the housing pocket 5 of the elastic containment exoskeleton 4 applied anatomically at the area of the user's body who, for example, is affected by the tumor, and is powered by a system connected to the power supply and an amplifier.

The reduced power applied by the medical, physiotherapeutic and/or protective device 1 of the present invention allows to perform the applications controlled by the system to which the data are sent by the attending physician, in terms of frequency, temperature, duration of exposure.

The technology uses the asymmetry in the distribution of negative and positive electric charges at the atomic or molecular level, leading to the formation of electric dipoles which tend to maintain alignment with any external electric field applied.

In presence of an alternating field, these dipoles rotate continuously, with the consequent conversion of part of the energy of the applied field into heat: this phenomenon goes under the name of "dielectric heating" which allows to operate on surface tumors (liver, kidneys, bones) and to penetrate tissues up to 1.5 cm deep.

The automatic management system of the wearable medical, physiotherapeutic and/or protective device 1 of the invention consists of:
- a heat sensing probe, useful to avoid excessive induced temperatures and thus produce ineffective or harmful operating conditions (in those points where there is a high presence of important blood vessels, temperature control is essential in order not to induce skin or vascular damage);
- a thermal probe (thermocouple) for detecting the temperature on the skin and managing the temperature to be emitted;
- a probe for detecting biometric data;
- a probe for radiofrequency emission for deep hyperthermia;
- a direct (or remote) control or adjustment of the temperature: a temperature range is programmed with respect to the estimated optimal temperature, this due to the different cutaneous heat sensitivity of different users. The temperature range is such as not to cause nuisance or damage even at the maximum expected level;
- medical Bluetooth communication system for remote management of the temperature, the radio frequency emitted, the duration of the application, and for the storage of the detected data (pressure, temperature before and after application, heart rate);
- smartphone or smartwatch application for the management of the wearable medical, physiotherapeutic and/or protective device 1 of the invention and of the detected data;
- this application is preferably open to sending the data detected to the attending physician, who may make changes on the timing and duration of the thermotherapeutic applications to be performed with the wearable medical, physiotherapeutic and/or protective device 1 of the invention.

Therefore, the description just provided allows to highlight how the wearable thermotherapeutic, medical, physiotherapeutic and/or protective device of the present invention achieves the purposes and reaches the advantages listed above.

The wearable medical, physiotherapeutic and/or protective device of the invention allows to perform a high-precision localized thermotherapy on the body of a person, is adapted to be used wherever possible, even during moments of relaxation outdoors, and is effective in the treatment of osteoarthritis and some forms of surface tumors.

The possibility of carrying out several daily applications leads to a physiological improvement such as to advantageously reduce the quantity of drugs which a person with physical or health problems must take.

The type of thermotherapy allowed by the wearable radiant, medical, physiotherapeutic and/or protective device of the invention is also applicable to remedy common diseases of a person, and therefore, for example for:
- the aesthetic recovery of skin;
- the absorption of muscle trauma;
- the improvement of blood thinning;
- the increase in body oxygenation;
- the normalization of blood pressure;
- the warming and relaxation of the body;
- the improvement of excess fluid drainage in the human body;
- the reduction of water retention;
- the improvement of sleep quality;
- the substantial elimination of lactic acid formation;
- the contrast of psycho-physical stress;
- the reduction of fatigue and tiredness.

The wearable radiant, medical, physiotherapeutic and/or protective device of the invention allows particularly, preferably but advantageously, to fully exploit the potential of heating (or radiant) means such as long infrared rays (FIR) that are useful to modify the state of the water inside our body, making it more "efficient" in its role as universal solvent able to:
- delivering nutrients to cells through the cell membrane;
- extracting the metabolism waste substances from the cells, also called waste products or acid toxins;
- improving peripheral vascularization;
- carrying out an adjuvant action for pharmacological therapies, better conveyed towards the part to be treated.

In fact, in order to achieve even only part of these relevant benefits, the wearable medical, physiotherapeutic and/or protective device of the invention:
- is precisely modeled on the anatomical part to be treated, due to its essential elements which form the basic structure thereof and which are elastically yielding;
- preferably uses a flexible and elastically yielding element as heating means (radiating heat by means of an infrared electromagnetic radiation), such as a flexible film, with a specific shape, frequencies and temperatures for each anatomical part to be treated;
- preferably provides operating parameters such as temperature, frequency and duration of thermotherapy, specifically defined for each anatomical part to be treated (for example, depending on the vascularization and thickness of the skin), for cutaneous heat sensitivity and/or age;
- preferably provides for the possibility, through adjustment means managed by the central processing and control unit, to adjust the temperature, in the range defined for the therapy, in order to produce the expected physiological effect, preventing disturbances due to excess heat or inefficiencies due to typical heat insufficiency of single temperature systems.

During the execution step, modifications may be made to the wearable radiant, medical, physiotherapeutic and/or protective device of the invention, consisting, for example, in more than one housing pocket in the elastic exoskeleton, in each of which at least one flexible film emitting electromagnetic radiation is housed.

The wearable medical, physiotherapeutic and/or protective device of the invention may also be used as a thermo-protective heating component, inside technical mountain or diving clothing.

In addition to this, there may be other constructive variants of the wearable medical, physiotherapeutic and/or protective device exclusively claimed herein, not illustrated in the attached tables, in which the single housing pocket houses a plurality of flexible films emitting electromagnetic radiation, each of which is suitably isolated from the other from an electrical point of view, which does not affect the overall advantage provided by the present invention.

It is apparent that many other variants may be made to the wearable medical and/or physiotherapeutic device in question, without thereby departing from the principles of novelty inherent in the inventive idea, just as it is apparent that in the practical implementation of the invention, the materials, shapes and sizes of the details shown may be any and may be replaced with other technically equivalent ones.

Where the constructive features and techniques mentioned in any successive claims are followed by reference signs or numerals, such reference signs were introduced for the sole purpose of increasing intelligibility of the claims themselves and consequently, such reference signs have no limiting effect on the interpretation of each element identified by way of example only by such reference signs.

## Claims

1. A wearable medical, physiotherapeutic and/or protective device (1; 50; 100; 150) comprising:
- a laminar body (2) which normally, in conditions of non-use of said medical, physiotherapeutic and/or protective device, assumes a substantially flat shape and which, at least in conditions of use of said medical, physiotherapeutic and/or protective device coinciding with the situation in which said medical, physiotherapeutic and/or protective device is worn by a patient, assumes a shape which follows or adapts to a localized anatomical area, to be subjected to heat therapy, of the human body of said patient;
- heating means (3) coupled to said laminar body (2) and adapted to be activated when said laminar body (2) is worn by said patient to act on said localized anatomical area of said human body to be subjected to said heat therapy,
**characterized in that** said laminar body (2) consists of an elastic containment exoskeleton (4) in which at least one housing pocket (5) is identified which houses at least one flexible film (6) emitting electromagnetic radiation, forming said heating means (3) and electrically connected to an electric power supply source (7), adapted to be arranged close to said localized anatomical area to be subjected to said heat therapy.

2. A device (1; 50; 100; 150) according to claim 1), **characterized in that** said flexible film (6) is removably coupled to said elastic containment exoskeleton (4).

3. A device (1; 50; 100; 150) according to claim 1) or 2), **characterized in that** said flexible film (6) is substantially arranged in the central part (4a) of said elastic containment exoskeleton (4), since said housing pocket (5) is obtained in said central part (4a) of said elastic containment exoskeleton (4).

4. A device (1; 50; 100; 150) according to any of the preceding claims, **characterized in that** said flexible film (6) is of the infrared type and emits infrared thermal radiation (IR).

5. A device (1; 50; 100; 150) according to claim 4), **characterized in that** said infrared thermal radiations are of long type, in class C, and have a frequency in the range 2÷30 µm.

6. A device (1; 50; 100; 150) according to any of the preceding claims, **characterized in that** said flexible film (6) emitting electromagnetic radiation comprises a hybrid printed electric circuit, made partially of a first electrically conductive material and partially of a second electrically insulating material so that said printed electric circuit is made of an overall material which can be defined as a semiconductor.

7. A device (1; 50; 100; 150) according to any of the preceding claims, **characterized in that** said elastic containment exoskeleton (4) is made of antibacterial polymeric material for printed medical use.

8. A device (1; 50; 100; 150) according to any of the preceding claims, **characterized in that** said elastic containment exoskeleton (4) comprises a base reticular structure (8) and a covering reticular structure (9) stably coupled to each other through joining means so as to define a free peripheral section in which an access mouth is identified which communicates with said housing pocket (5) and through which said flexible film (6) emitting electromagnetic radiation is introduced into said housing pocket (5).

9. A device (1; 50; 100; 150) according to any of the preceding claims, **characterized in that** said elastic containment exoskeleton (4) is anatomically shaped both as a lateral profile and as a graphic design, so as to follow the muscle fascia of said localized anatomical area of said human body to be subjected to said heat therapy.

10. A device (1; 50; 100; 150) according to any of the preceding claims, **characterized in that** said electric power supply source (7) comprises a battery (10) housed in a surface seat (11) obtained in said elastic exoskeleton containment (4).

11. A device (1; 50; 100; 150) according to any of the preceding claims, **characterized in that** said electric power supply source (7) comprises a battery (10) consisting of a plurality of mini-accumulators having an electric voltage in the range 5÷9 Volts and an electric current no greater than 5 ampere hours (Ah).

12. A device (1; 50; 100; 150) according to any of the preceding claims, **characterized in that** said heating means (3) also comprise at least one radiofrequency emitting plate or head (12) adapted to be arranged close to said localized anatomical area of said human patient's body to be subjected to said heat therapy, coupled to said elastic containment exoskeleton (4) and electrically connected to said electric power supply source (7).

13. A device (1; 50; 100; 150) according to claim 12), **characterized in that** a radiofrequency emitting plate or head (12) is removably coupled to said elastic containment exoskeleton (4) by means of snap coupling means.

14. A device (1; 50; 100; 150) according to any of the preceding claims, **characterized in that** it comprises one or more operating sensors, coupled to said elastic exoskeleton (4) and electrically connected to said electric power supply source (7), adapted to detect the vital values of said human body of said patient.

15. A device (1; 50; 100; 150) according to any of the preceding claims, **characterized in that** it comprises a central processing and control unit, electrically connected to said electric power supply source (7), adapted to manage the operating parameters of said heating means (3) following commands transmitted by a user and to store the vital data of said patient of which said localized anatomical area of said human body is subjected to said heat therapy.
